# EUROPÄISCHE PATENTANMELDUNG

(11) **EP 3 363 349 A1**
(43) Veröffentlichungstag der Anmeldung: **22.08.2018**
(21) Anmeldenummer: 17172357.0
(22) Anmeldetag: 23.05.2017
(51) Int. Cl.: A61B 5/00, H04B 10/80, A61B 5/0205, A61B 5/0428

(54) **MESSEINRICHTUNG ZUR BESTIMMUNG PHYSIOLOGISCHER MESSWERTE EINES PATIENTEN UND SENSOREINHEIT**

(30) Priorität: 25.08.2016 DE 102016010428
(71) Anmelder: seca ag, 4153 Reinach BL 1 (CH)
(72) Erfinder: Vogel, Frederik, 22301 Hamburg (DE)
(74) Vertreter: Klickow & Wetzel PartGmbB

(57) **Zusammenfassung**

Es wird eine Messeinrichtung zur Bestimmung physiologischer Messwerte eines Patienten vorgestellt mit einer grafischen Benutzerschnittstelle, einer Steuer- und Auswerteeinheit, einer Sensorverbindungseinheit, sowie einem oder mehreren Sensoren zur Aufnahme vorbestimmter physiologischer Messwerte, wobei die Sensoren mit der Sensorverbindungseinheit mittels separater serieller Sensorschnittstellen verbunden sind, und die Sensorverbindungseinheit mit der Steuer- und Auswerteeinheit mittels einer seriellen Steuerungsschnittstelle verbunden ist. Das Messsystem zeichnet sich dadurch aus, dass eine oder mehrere der Sensorschnittstellen als galvanische getrennte Schnittstellen ausgeführt sind.

Weiterhin wird eine Sensoreinheit eines entsprechenden Messsystems vorgestellt.

## Beschreibung

Die Erfindung betrifft eine Messeinrichtung zur Bestimmung physiologischer Messwerte eines Patienten mit einer grafischen Benutzerschnittstelle, einer Steuer- und Auswerteeinheit, einer Sensorverbindungseinheit, sowie einem oder mehreren Sensoren zur Aufnahme vorbestimmter physiologischer Messwerte, wobei die Sensoren mit der Sensorverbindungseinheit mittels separater serieller Sensorschnittstellen verbunden sind, und die Sensorverbindungseinheit mit der Steuer-und Auswerteeinheit mittels einer seriellen Steuerungsschnittstelle verbunden ist.

In der klinischen Praxis ist es üblich, bestimmte physiologische Messwerte vom Patienten, die auch als Vitaldaten bezeichnet werden, standardmäßig zu bestimmen. Zu diesen Messwerten gehören beispielsweise der Blutdruck, die Pulsrate, die Körpertemperatur und die arterielle Sauerstoffsättigung. Für die einzelnen Messwerte sind von verschiedenen Hersteller Sensoren erhältlich, welche die Messwerte i.d.R. nichtinvasiv ermitteln und über eine zumeist als serielle Schnittstelle ausgeführte Datenschnittstelle bereitstellen. Je nach Gepflogenheit des Benutzers werden bestimmte Sensoren kombiniert und die entsprechenden Messwerte gemeinsam erfasst und über eine Benutzerschnittstelle zur Verfügung gestellt.

Sowohl die beschriebenen Messsysteme als auch die entsprechenden Sensoren stellen unabhängig voneinander Medizingeräte dar, deren elektrische Betriebssicherheit vom Hersteller garantiert werden muss. Zusätzlich muss der Hersteller eines Messsystems, welches mit unterschiedlichen Sensoren betrieben werden kann, die Betriebssicherheit für jede mögliche Kombination von angeschlossenen Sensoren garantieren. Dies wird insbesondere dann schwierig, wenn die entsprechenden Sensoren von anderen Herstellern stammen.

Es besteht daher die Aufgabe der Erfindung darin, ein Messsystem zur Bestimmung physiologischer Messwerte eines Patienten bereitzustellen, welches in Hinsicht auf diese Problematik verbessert ist.

Diese Aufgabe wird gemäß der Erfindung gelöst durch eine Messeinrichtung zur Bestimmung physiologischer Messwerte eines Patienten mit einer grafischen Benutzerschnittstelle, einer Steuer- und Auswerteeinheit, einer Sensorverbindungseinheit, sowie einem oder mehreren Sensoren zur Aufnahme vorbestimmter physiologischer Messwerte, wobei die Sensoren mit der Sensorverbindungseinheit mittels separater serieller Sensorschnittstellen verbunden sind, und die Sensorverbindungseinheit mit der Steuer- und Auswerteeinheit mittels einer seriellen Steuerungsschnittstelle verbunden ist, welches dadurch weitergebildet sein kann, dass eine oder mehrere der Sensorschnittstellen als galvanische getrennte Schnittstellen ausgeführt sind.

Unabhängig von der jeweiligen elektrischen Konfiguration einzelner Sensoren wird durch die galvanische Trennung der Schnittstellen gewährleistet, dass keine die Betriebssicherheit beeinträchtigenden Patientenströme zwischen dem Sensor und dem Messsystem fließen können. Die galvanische Trennung kann beispielsweise durch den Einsatz optischer Schnittstellen und/oder den Einsatz galvanisch getrennter Gleichstromwandler (DC/DC) erfolgen.

In einer möglichen Ausführung des Messsystems sind die Sensorschnittstellen als RS232-Schnittstellen ausgeführt. RS232-Schnittstellen sind weit verbreitet und zur Kommunikation mit einfachen Sensoren besonders gut geeignet.

In einer bevorzugten Weiterbildung des Messsystems ist die Steuerungsschnittstelle als USB-Schnittstelle ausgeführt. USB-Schnittstellen sind zwar aufwändiger als RS232-Schnittstellen, jedoch für komplexe Kommunikationsvorgänge besser geeignet.

In einer möglichen Umsetzung des Messsystems können eine oder mehrere der Sensorschnittstellen als galvanisch nicht getrennte Schnittstellen ausgeführt sein. Dies ist insbesondere dann vorteilhaft, wenn die über die entsprechenden Schnittstellen angeschlossenen Sensoren vom gleichen Hersteller stammen wie das Messsystem, so dass die Betriebssicherheit durch ein aufeinander abgestimmtes elektronisches Design gewährleistet werden kann, ohne dass eine galvanische Trennung erforderlich ist.

In einer Ausführung des Messsystems weist die Steuer- und Auswerteeinheit wenigstens eine Schnittstelle zum direkten Anschluss weiterer Sensoren auf. Dies ist insbesondere dann interessant, wenn komplexe Sensoren in das Messsystem eingebunden werden sollen, welche nicht über eine serielle Schnittstelle angesteuert werden können. Hierzu gehören beispielsweise Bioelektrische Impedanzsensoren (BIA).

Das Messsystem kann derart gestaltet sein, dass die grafische Benutzerschnittstelle und die Steuer- und Auswerteeinheit in einem Steuerungsgehäuse angeordnet sind, und dass die Sensorverbindungseinheit in einem Sensorgehäuse angeordnet ist. Beispielsweise kann die Benutzerschnittstelle ein berührungsempfindliches Display (Touchscreen) umfassen, auf welchem grafische Bedien- und/oder Anzeigeelemente dargestellt werden können. Die Steuer- und Auswerteeinheit kann in durch programmtechnische Mittel auf einer Standard-PC-Hardware ausgeführt sein. Die PC-Hardware und das berührungsempfindliche Display können als medizintechnisch zugelassener PC bereitgestellt werden. Durch die Unterbringung der Sensorverbindungseinheit in einem separaten Gehäuse ist eine technische Modifikation des PCs nicht erforderlich. Dabei können neben der Sensorverbindungseinheit auch weitere, sensorspezifische Komponenten in dem Sensorgehäuse angeordnet sein.

In einer bevorzugten Ausführung des Messsystems weisen das Steuerungsgehäuse und/oder das Sensorgehäuse Verbindungsmittel auf, um die Gehäuse lösbar miteinander zu verbinden, und die Verbindung zwischen der Steuer- und Auswerteeinheit und der Sensorverbindungseinheit ist als Steckverbindung ausgeführt. Bei den Verbindungselementen kann es sich um einfache Haken oder andere geeignete Elemente handeln. Die Steckverbindung ist vorteilhafterweise als Standard-USB-Stecker ausgeführt, welcher mit einem kurzen Kabel am Sensorgehäuse befestigt ist und in eine USB-Buchse des Steuergehäuses eingeführt ist bzw. wird. Anstelle eines Standard-USB-Steckers kann auch ein Mini-USB- oder Mikro-USB-Stecker verwendet werden.

Das Sensorgehäuse des Messsystems kann mechanische Aufnahmemittel zur Aufnahme eines oder mehrerer Sensoren aufweisen. Dadurch wird das Messsystem zusammen mit den zugehörigen Sensoren als kompakte Einheit besonders leicht handhabbar.

Vorzugsweise sind in einem entsprechenden Messsystem die mechanischen Aufnahmemittel zur Aufnahme spezifischer Sensortypen ausgeführt, und die Sensorverbindungseinheit zur Kommunikation mit denselben spezifischen Sensortypen konfiguriert. Die Vielzahl von am Markt verfügbaren Sensoren unterschiedlicher Hersteller, welche unterschiedliche mechanische Gehäuse aufweisen, können nur schwierig in den mechanischen Aufnahmemitteln aufgenommen werden. Gleichzeitig verwenden unterschiedliche Sensoren teilweise unterschiedliche Kommunikationsprotokolle, welche von der Sensoranschlusseinheit bedient werden müssen. Durch die Einschränkung auf bestimmte Sensortypen können passende Aufnahmemittel bereitgestellt und die benötigten Kommunikationsprotokolle in der Sensoranschlusseinheit vorinstalliert werden.

In einer weiter bevorzugten Ausführung des Messsystems ist die Steuer- und Auswerteeinheit eingerichtet, die Konfiguration der Sensorverbindungseinheit zu ermitteln und die grafische Benutzerschnittstelle so anzusteuern, dass nur mit der vorliegenden Konfiguration kompatible Eingaben und/oder Ausgaben möglich sind. Die Steuer- und Auswerteeinheit erkennt also, welche Sensortypen an das Messsystem angeschlossen sind bzw. werden können, und stellt die entsprechenden Anzeige- und Eingabeelemente an der grafischen Benutzerschnittstelle zur Verfügung. Solche Anzeige- oder Eingabeelemente, die in der jeweiligen Konfiguration nicht benötigt werden, können hingegen ausgeblendet werden.

In einer besonders bevorzugten Ausführung eines Messsystems ist dieses als modulares Messsystem ausgeführt ist, welches eine erste Sensoreinheit mit einer ersten Sensorverbindungseinheit in einem ersten Sensorgehäuse und eine erste Gruppe von Sensoren, sowie eine zweite Sensoreinheit mit einer zweiten Sensorverbindungseinheit in einem zweiten Sensorgehäuse und eine zweiten Gruppe von Sensoren umfasst, wobei die Benutzerschnittstelle und die Steuer- und Auswerteeinheit wahlweise mit der ersten Sensoreinheit oder mit der zweiten Sensoreinheit zu einem Messsystem verbunden werden kann. Es können somit Sensoreinheiten mit Gruppen von Sensoren bereitgestellt werden, die von Benutzern häufig miteinander verwendet werden. Der Konfigurationsaufwand für den Benutzer wird dabei deutlich reduziert.

Die Aufgabe wird ferner gelöst durch eine Sensoreinheit eine Messeinrichtung gemäß den obigen Ausführungen. Bezüglich der hierdurch erreichbaren Vorteile wird ausdrücklich auf das oben gesagte verwiesen.

Die Erfindung wird im Folgenden anhand einiger exemplarischer Darstellungen verdeutlicht. Es zeigen:
- Figur 1:: den prinzipiellen Aufbau eines Messsystems,
- Figur 2a-2e:: unterschiedliche Benutzeroberflächen eines Messsystems,
- Figur 3:: den prinzipiellen Aufbau eines weiteren Messsystems,
- Figur 4:: den prinzipiellen Aufbau einer Sensoranschlusseinheit mit mehreren Schnittstellen,
- Figur 5a, 5b:: ein weiteres Messsystem Vorder- und Rückansicht,
- Figur 6a, 6b:: ein zusätzliches Messsystem Vorder- und Rückansicht,
- Figur 7:: ein Sensorgehäuse in einer partiellen Explosionsdarstellung.

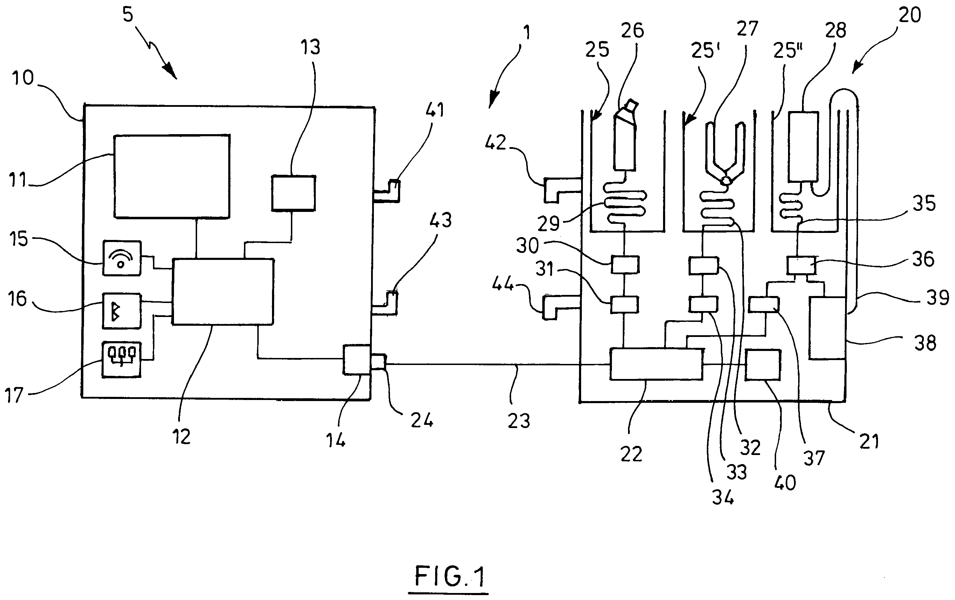

Figur 1 zeigt den prinzipiellen Aufbau eines Messsystems 1. Das Messsystem 1 umfasst eine Steuerungseinheit 5 mit einem Steuerungsgehäuse 10, in welchem eine grafische Benutzerschnittstelle 11 in Form eines Touchscreens angeordnet ist. Dabei kann es sich beispielsweise um einen resistiv arbeitenden Touchscreen handeln. Weiterhin sind in dem Sensorgehäuse 10 eine Steuer- und Auswerteinheit 12, welche mit einem Speicherelement 13 sowie eine USB-Schnittstelle 14 verbunden ist, die beispielsweise nach dem USB 2.0 oder USB 3.0 Standard ausgeführt ist. Weiter können in dem Steuerungsgehäuse zusätzliche Schnittstellen wie eine WiFi-Schnittstelle 15, eine Bluetooth-Schnittstelle 16 und/oder eine Ethernet-Schnittstelle 17 vorgesehen und mit der Steuer- und Auswerteeinheit 12 verbunden sein.

Die Steuerungs- und Auswerteeinheit 12 kann als Single Board Computer (SBC) ausgeführt sein und beispielsweise auf einem Prozessor des Typs AM335x der Firma Texas Instruments basieren.

Das Messsystem 1 umfasst weiterhin eine Sensoreinheit 20 mit einem Sensorgehäuse 21. In dem Sensorgehäuse 21 ist eine Sensorverbindungseinheit 22 angeordnet, welche über ein Kabel 23 mit der USB-Schnittstelle 14 verbunden ist. Dazu ist am Ende des Kabels 23 ein USB-Stecker 24 vorgesehen, welcher als Standard-, Mini- oder Mikro-USB-Stecker ausgeführt sein kann. Die USB-Schnittstelle 14 weist eine entsprechende Steckbuchse auf.

In dem Sensorgehäuse 21 sind mehrere, im dargestellten Beispiel drei, mechanische Aufnahmen 25, 25', 25" zur Aufnahme verschiedener Sensoren 26, 27, 28 vorgesehen. Bei dem Sensor 26 handelt es sich im dargestellten Beispiel um ein Infrarotthermometer zum Messen der Körpertemperatur im Ohr eines Patienten. Der Sensor 26 ist mittels eines Kabels 29 mit einer sensorspezifischen Schaltung 30 verbunden, welche das Ausgangssignal des Sensors 27 in ein RS232-kompatibles Signal umsetzt. Die Schaltung 30 ist wiederum über eine RS232-Schnittstelle 31 mit der Sensorverbindungseinheit 22 verbunden. Der Sensor 27, bei dem es sich um einen als optische Fingerklemme ausgeführten Sauerstoffsättigungssensor handelt, ist auf gleiche Weise über ein Kabel, eine Schaltung 33 und eine RS232-Schnittstelle 34 mit der Sensorverbindungseinheit 22 verbunden. Die RS232-Schnittstelle 34 ist als galvanisch getrennte Schnittstelle ausgeführt. Sensor 28, bei dem es sich um einen nichtinvasiven Blutdrucksensor mit pneumatischer Kompressionsmanschette handelt, ist auf ähnliche Weise über ein Kabel 35, eine Schaltung 36 und eine RS232-Schnittstelle 37 mit der Sensorverbindungseinheit 22 verbunden. Dabei ist an die Schaltung 36 als weitere für den Sensor 28 spezifische Komponente ein Kompressor 38 vorgesehen, welcher die Kompressionsmanschette des Sensors 28 über einen Schlauch 39 aufpumpen und entlüften kann. Die RS232-Schnittstelle 37 ist ebenfalls als galvanisch getrennte Schnittstelle ausgeführt.

Bei den Schaltungen 30, 32, 36 handelt es sich um von den Herstellern der jeweiligen Sensoren 26, 27, 28 zur Verfügung gestellte OEM-Module. Auch wenn diese Module alle den verbreiteten RS232-Standard zur Datenübertragung verwenden, gibt es in der Regel für jeden der Sensoren 26, 27, 28 ein spezifisches übergeordnetes Protokoll. Informationen über die entsprechenden Protokolle sind in einem Speicherelement 40 hinterlegt, welcher mit der Sensorverbindungseinheit 22 verbunden ist. In dem Speicherelement 40 sind weiterhin Informationen übe die in der Sensoreinheit 20 vorgehaltenen Sensoren 26, 27, 28 abgelegt.

Das Steuerungsgehäuse 10 und das Sensorgehäuse 21 sind über form- und funktionskomplementäre Verbindungsmittel 41, 42, 43, 44 lösbar miteinander verbindbar, so dass die Steuerungseinheit 5 und die Sensoreinheit 20 gemeinsam gehandhabt werden können.

Die Arbeitsweise des dargestellten Messsystems 1 ist wie folgt: Bei Aktivierung des Messsystems 1 sendet die Steuer- und Auswerteeinheit 12 ein Abfragesignal über die USB-Schnittstelle 14 an die Sensorverbindungseinheit 22. Die Sensorverbindungseinheit lädt daraufhin Informationen über die in der Sensoreinheit 20 vorgehaltenen Sensoren 26, 27, 28 aus dem Speicherelement 40 und überträgt diese über die USB-Schnittstelle 14 an die Steuer- und Auswerteeinheit 12.

Die Steuer- und Auswerteeinheit 12 lädt dann aus dem Speicherelement 13 Informationen über für die Sensoren 26, 27, 28 anzuzeigenden Eingabe- und Ausgabeelemente, und erstellt auf Basis dieser Informationen eine auf der grafischen Benutzerschnittstelle 11 darzustellende Benutzeroberfläche, welches dann auf der grafischen Benutzerschnittstelle 11 dargestellt wird.

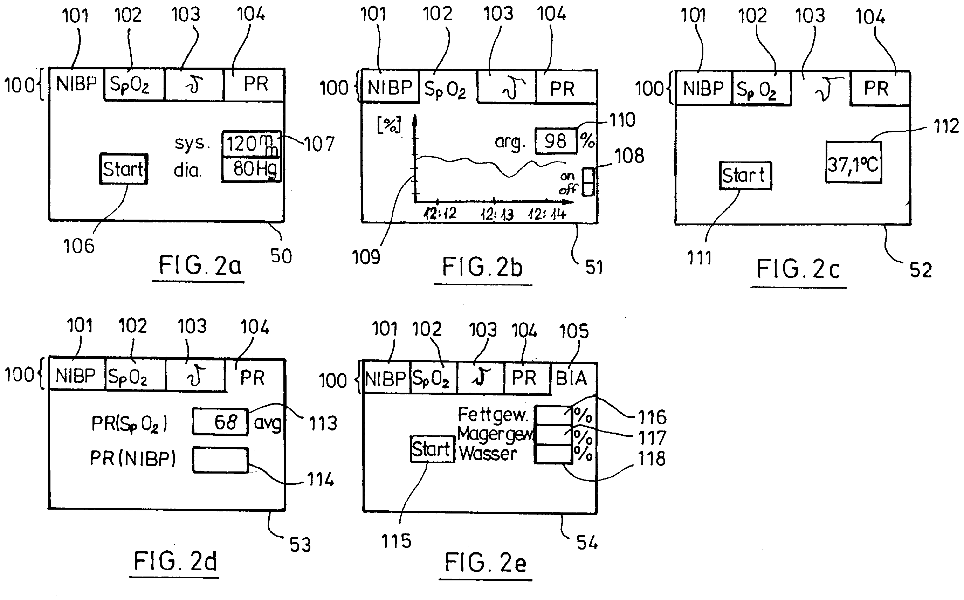

In den Figuren 2a bis 2e sind unterschiedliche Benutzeroberflächen 50, 51, 52, 53, 54 dargestellt. Diese weisen alle im oberen Bereich eine Reiterleiste 100 auf, in welcher einzelne Reiter 101, 102, 103, 104,105 dargestellt sind. Auf den Reitern 101, 102, 103, 104, 105 sind Bezeichnungen für verschiedene Messwerte dargestellt, nämlich "NIBP" für den Blutdruck, "SpO2" für die arterielle Sauerstoffsättigung, "ϑ" für die Körpertemperatur und "PR" für die Pulsrate. Auf dem Reiter 105 ist die Bezeichnung "BIA" für die Bioelektrische Impedanzanalyse abgebildet.

Figur 2a zeigt eine Benutzeroberfläche 50 zur Messung des Blutdrucks, die mittels des Sensors 28 durchgeführt wird. Der Benutzer gelangt auf diese Oberfläche durch Berühren des Reiters 101.

Die Blutdruckmessung mittels einer Kompressionsmanschette umfasst verschiedene Schritte und kann nicht kontinuierlich erfolgen. Daher ist auf der entsprechenden Benutzeroberfläche 50 eine Schaltfläche 106 vorgesehen, welche einen Startknopf darstellt. Durch Berühren der Schaltfläche 106 wird der Messvorgang gestartet. Dazu gibt die Steuer- und Auswerteeinheit 12 ein Anforderungssignal über die USB-Schnittstelle 14 an die Sensoranschlusseinheit 22, welche es über die RS232-Schnittstelle 37 an die Schaltung 36 weiterleitet. Die Schaltung 36 steuert dann den Kompressor 38 und in der Kompressionsmanschette 28 vorgesehene Messwertaufnehmer in der für die Messung erforderlichen Abfolge an. Nach Abschluss der Messung sendet die Schaltung das Messergebnis über die RS232-Schnittstelle 37 an die Sensoranschlusseinheit 22, welche es über die USB-Schnittstelle 14 an die Steuer- und Auswerteeinheit 12 weiterleitet. Das Ergebnis wird dann in dem Anzeigeelement 107 der Benutzeroberfläche 50 dargestellt.

Alternativ oder zusätzlich zu der Schaltfläche 106 kann an dem Sensor 28 ein nicht dargestellter Aktivierungsschalter vorgesehen sein, welcher ebenfalls den Messvorgang auslöst. In diesem Fall wird nur das Messergebnis über die RS232-Schnittstelle 37 an die Sensoranschlusseinheit 22, welche es über die USB-Schnittstelle 14 an die Steuer- und Auswerteeinheit 12 weiterleitet. Das Ergebnis wird wiederum in dem Anzeigeelement 107 der Benutzeroberfläche 50 dargestellt.

Bei Auslösung des Messvorgangs über einen Aktivierungsschalter am Sensor 28 wird nicht zwangsläufig die zu dem Sensor 28 gehörende Benutzeroberfläche 50 auf der grafischen Benutzerschnittstelle 11 dargestellt. Die Steuer- und Auswerteschaltung kann jedoch die entsprechende Benutzeroberfläche 50 eigenständig darstellen, sobald sie entsprechende Messergebnisse vom Sensor 28 erhält. Alternativ kann das Ergebnis auch in dem Anzeigeelement 107 eingetragen werden, ohne dass die entsprechende Benutzeroberfläche 50 dargestellt wird. Der Benutzer kann das Ergebnis dann erst sehen, wenn er die entsprechende Benutzeroberfläche 50 manuell auswählt.

Figur 2b zeigt eine Benutzeroberfläche 51 zur Messung der kapillaren Sauerstoffsättigung, zu welcher der Benutzer durch Berühren des Reiters 102 gelangt. Die Messung der Sauerstoffsättigung erfolgt über den Sensor 27, bei dem es sich um eine optische Fingerklemme handelt.

Der Sensor 27 kann kontinuierlich Messwerte liefern. Daher ist auf der Benutzeroberfläche 51 ein Schaltelement 108 vorgesehen, mittels dessen eine kontinuierliche Messung durch den Sensor 27 aktiviert und deaktiviert werden kann. Wird die Messung aktiviert, so sendet die Steuer- und Auswerteeinheit 12 ein Aktivierungssignal über die USB-Schnittstelle 14 an die Sensoranschlusseinheit 22, diese leitet es über die RS232-Schnittstelle 34 an die Schaltung 33 weiter. Nach Empfang des Aktivierungssignals ermittelt die Schaltung 33 in regelmäßigen Abständen, beispielsweise einmal pro Sekunde, einen Messwert für die arterielle Sauerstoffsättigung, und überträgt diesen über die RS232-Schnittstelle 33 an die Sensoranschlusseinheit22, welche die Messwerte über die USB-Schnittstelle 14 an die Steuer- und Auswerteinheit 12 überträgt. Die Messwerte werden auf der Benutzeroberfläche 51 auf einem Anzeigeelement 109 dargestellt, welches eine zeitliche Verlaufskurve abbilden kann. Zusätzlich kann die Steuer- und Auswerteeinheit 12 einen Mittelwert der Messwerte in einem bestimmten Zeitraum ermitteln und diesen in einem weiteren Anzeigeelement 110 darstellen.

Wie oben beschrieben können auch vom Sensor 27 Messwerte geliefert werden, wenn die Benutzeroberfläche 51 nicht auf der grafischen Benutzerschnittstelle 11 angezeigt wird. In diesem Fall werden die Anzeigeelemente 109 und 110 trotzdem aktualisiert, dies wird aber erst nach manuellem Umschalten auf die Benutzerschnittstelle 51 sichtbar.

Figur 2c zeigt eine Benutzeroberfläche 52 zur Messung der Körpertemperatur, zu welcher der Benutzer durch Berühren des Reiters 103 gelangt. Die Messung erfolgt über den Sensor 16.

Der Aufbau der Benutzeroberfläche 52 ähnelt dem der Benutzeroberfläche 50. Auch hier gibt es eine Schaltfläche 111 zur Auslösung des Messvorgangs sowie ein Anzeigeelement 112 zur Darstellung des Messergebnisses. Der Kommunikationsablauf zwischen der Steuer- und Auswerteeinheit 12 und der sensorspezifischen Schaltung 30 entspricht ebenfalls dem zur Figur 2a beschriebenen Ablauf.

Wenn anstelle eines IR-Thermometers ein Kontaktthermometer zur axillaren, oralen oder rektalen Anwendung als Temperatursensor in dem Messsystem 1 vorgesehen ist, kann dieser Sensor auch kontinuierliche Messwerte liefern. In diesem Fall kann die Benutzeroberfläche 52 ähnlich der Benutzeroberfläche 51 ausgeführt sein, und der Kommunikationsablauf mehr dem zur Figur 2b beschriebenen Ablauf entsprechen.

In Figur 2d ist eine Benutzeroberfläche 53 zur Messung der Pulsrate dargestellt, zu welcher der Benutzer durch Berühren des Reiters 104 gelangt. Anders als oben beschriebenen Messungen ist für die Messung der Pulsrate kein eigener Sensor vorgesehen. Sowohl der Sensor 28 als auch der Sensor 27 liefern automatisch einen Messwert für die Pulsrate mit. Daher ist auf der Benutzeroberfläche 53 ein Anzeigeelement 113 vorgesehen, in welchem die mittels des Sensors 27 gemessene Pulsrate angezeigt wird, und es ist ein Anzeigeelement 114 vorgesehen, in welchem die mittels des Sensors 28 gemessene Pulsrate dargestellt wird. Wenn von einem der Sensoren noch kein Messwert vorliegt, so ist das entsprechende Anzeigeelement leer.

Da es sich bei der Pulsratenmessung um eine sekundäre Messung, also um eine Messung ohne eigenen Sensor, handelt, ist auf der Benutzeroberfläche 53 keine Schaltfläche zur Aktivierung der Messung vorgesehen.

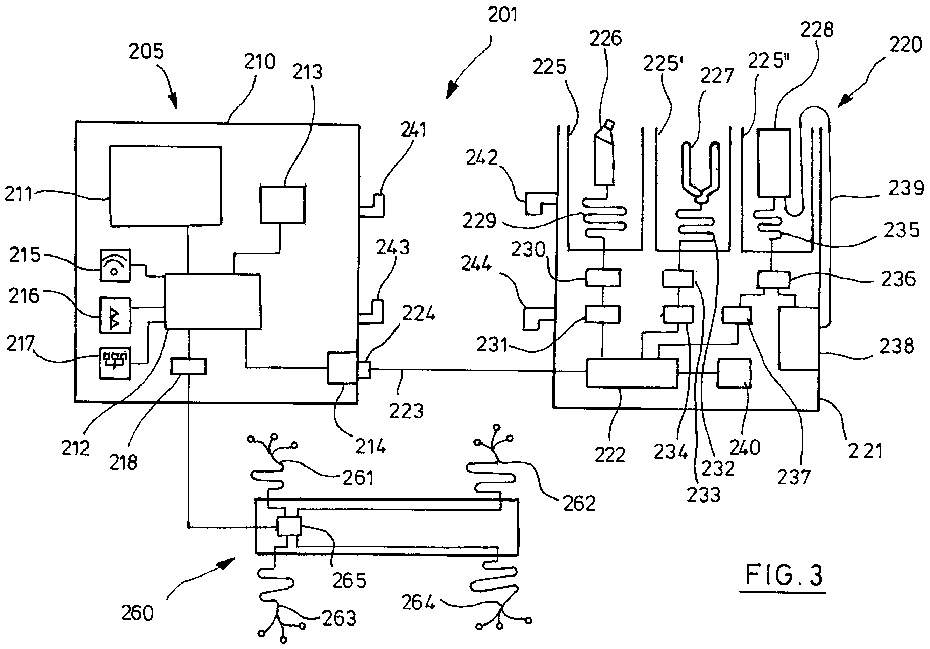

In Figur 2e ist eine Benutzeroberfläche 54 eines Messsystems dargestellt, welches mit einem zusätzlichen Sensor für ein Bioelektrische Impedanzanalyse (BIA) konfiguriert ist. Ein entsprechendes Messsystem ist in Figur 3 dargestellt und wird weiter unten beschrieben. Bei der BIA werden mittels elektrischen Ströme die Anteile verschiedener Bestandteile an der Gesamtmasse eines Patienten bestimmt, insbesondere die Anteile von Fettgewebe, Magergewebe und Wasser. Die Benutzeroberfläche 54 wird durch Berühren des Reiters 105 aktiviert.

Die BIA erfordert ein komplexes Messverfahren, weswegen ähnlich wie bei der Blutdruckmessung keine kontinuierliche Messung möglich ist. Daher ist auf der Benutzeroberfläche 54 eine Schaltfläche 115 vorgesehen, bei deren Berühren der Messvorgang ausgelöst wird. Weiterhin sind Anzeigeelemente 116, 117, 118 vorgesehen, in denen das Messergebnis in Form der Anteile von Fettgewebe, Magergewebe und Wasser an der Gesamtmasse des Patienten dargestellt wird.

Figur 3 zeigt ein Messsystem 201, welches zur Durchführung einer BIA konfiguriert ist. Der Aufbau des Messsystems 201 entspricht in weiten Teilen dem des in Figur 1 dargestellten Messsystems 1. Sich in Struktur und Funktion entsprechende Komponenten sind daher in Figur 3 mit einem um 200 erhöhten Bezugszeichen versehen und werden nicht weiter erläutert.

Im Gegensatz zum Messsystem 1 ist in dem Messsystem 201 eine spezielle Schnittstelle 218 vorgesehen, welche direkt mit der Steuer- und Auswerteeinheit 212 verbunden ist. Bei der Schnittstelle 218 handelt es sich um eine proprietäre binäre Schnittstelle, welche für die komplexe Kommunikation mit einem BIA-Sensor 260 eingerichtet ist. Der BIA-Sensor 260 umfasst mehrere Gruppen von Messelektroden 261, 262, 263, 264, welche von einer Steuerung 265 versorgt werden. Die Steuerung 265 ist über die Schnittstelle 218 mit der Steuer- und Auswerteeinheit 212 verbunden.

Löst nun der Benutzer durch Berühren der Schaltfläche 115 auf der Benutzeroberfläche 54 eine Messung aus, so sendet die Steuer- und Auswerteeinheit ein Anforderungssignal über die Schnittstelle 218 an die Steuerung 265. Die Steuerung 265 leitet daraufhin verschiedene Messströme über die Messelektroden 261, 262, 263, 264 durch den Patienten und misst dabei verschiedene elektrische Parameter, welche wiederum über die Schnittstelle 218 an die Steuer- und Auswerteeinheit 212 übertragen werden. In der Steuer- und Auswerteeinheit 212 werden daraus mittels im Speicherelement 213 hinterlegter Rechenregeln die Anteile von Fettgewebe, Magergewebe und Wasser an der Gesamtmasse des Patienten bestimmt. Dazu kann zusätzlich auf physiologische Daten des Patienten wie Alter, Geschlecht, Körpergröße etc. zurückgegriffen werden, welche entweder vorab über die grafische Benutzerschnittstelle 211 eingegeben werden, oder mittels einer der Schnittstellen 215, 216, 217 aus einer Patientendatenbank geladen werden.

Bei der Steuerungseinheit 5, 205 kann es sich um einen als Medizingerät zugelassenen PC handeln, der durch im Speicherelement 13, 213 hinterlegte programmtechnische Mittel eingerichtet ist, die beschriebenen Funktionen auszuführen. In diesem Fall kann die Sensoreinheit 20, 220 als separate Zusatzeinheit angeboten werden, um mit dem PC zu dem beschriebenen Messsystem 1, 201 kombiniert zu werden. Je nach Gepflogenheiten des Anwenders kann dazu die Sensoreinheit 20, 220 mit unterschiedlichen Sensortypen konfiguriert sein.

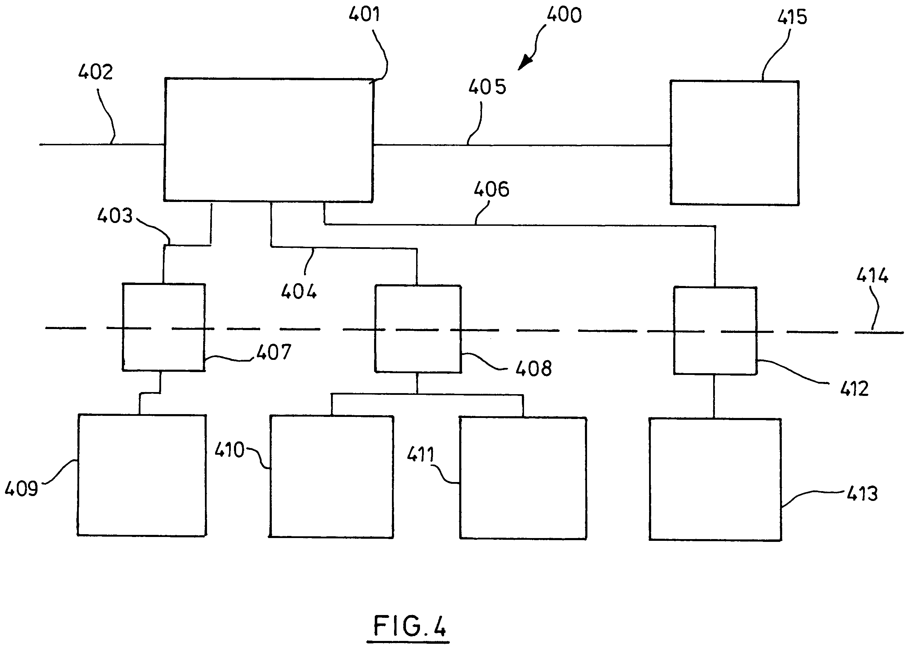

In Figur 4 ist ein möglicher Aufbau einer Sensorverbindungseinheit 400 mit mehreren Schnittstellen dargestellt. Die Sensorverbindungseinheit 400 umfasst einen USB-RS232-Umsetzer 401, welcher über eine Leitung 402 mit einer USB-Schnittstelle einer Steuerungseinheit verbunden werden kann. Der USB-RS232-Umsetzer 401 arbeitet als bidirektionaler Multiplexer/Demultiplexer, kann also ein von einer USB-Schnittstelle empfangenes Signal selektiv auf mehrere RS232-Schnittstellen weiterleiten, und von mehreren RS232-Schnittstellen empfangene Signale zu einem USB-Signal zusammenführen. Im dargestellten Beispiel hat der USB-RS232-Umsetzer 401 drei RS232-Anschlüsse 403, 404, 405 und einen Versorgungsspannungsausgang 406. Die RS232-Anschlüsse 403, 404 sind über Optokoppler 407, 408 mit Schnittstellen 409, 410, 411 verbunden, an welche sensorspezifische Schaltungen angeschlossen werden können. Dabei sind die Schnittstellen 410, 411 gemeinsam an den Optokoppler 408 angeschlossen, sie können daher nur alternativ zueinander genutzt werden. Der Versorgungsspannungsausgang 406 ist über einen galvanisch getrennten Gleichspannungswandler 412 mit einer Versorgungsspannungsschnittstelle 413 verbunden.

Die Schnittstellen 409, 410, 411 und 413 sind somit galvanisch vollständig von der Steuerungseinheit getrennt, somit ist die Patientensicherheit gegen Fehlerströme gesichert. Dies ist in der Figur 4 durch die gestrichelte Linie 414 angedeutet. Der RS232-Anschluss 405 ist ohne galvanische Trennung mit der Schnittstelle 415 verbunden. Bei an der Schnittstelle 414 anzuschließenden Sensoren muss somit die Patientensicherheit durch andere Maßnahmen gewährleistet werden.

Die beschriebenen Messsysteme können in unterschiedlichen Varianten mit verschiedenen Sensortypen konfiguriert werden, von denen einige exemplarisch in der unten stehenden Tabelle aufgelistet sind. Weitere, nicht aufgelistete Varianten sind ebenfalls möglich.

| Variante | NIBP | SpO2 | | Temperatur | | | BIA |
|---|---|---|---|---|---|---|---|
| | | Modell A | Modell B | oral/axillar | rektal | IR | |
| 1 | X | X | | X | | | X |
| 2 | X | X | | | X | | X |
| 3 | X | X | | | | X | X |
| 4 | X | | X | X | | | X |
| 5 | X | | X | | X | | X |
| 6 | X | | X | | | X | X |
| 7 | X | X | | | | | |
| 8 | X | | X | | | | |
| 9 | X | | | X | | | |
| 10 | X | | | | X | | |
| 11 | X | | | | | X | |
| 12 | X | X | | X | | | |
| 13 | X | X | | | X | | |
| 14 | X | X | | | | X | |
| 15 | X | | X | X | | | |
| 16 | X | | X | | X | | |
| 17 | X | | X | | | X | |
| 18 | X | X | | | | | X |
| 19 | X | | X | | | | X |
| 20 | X | | | X | | | X |
| 21 | X | | | | X | | X |
| 22 | X | | | | | X | X |

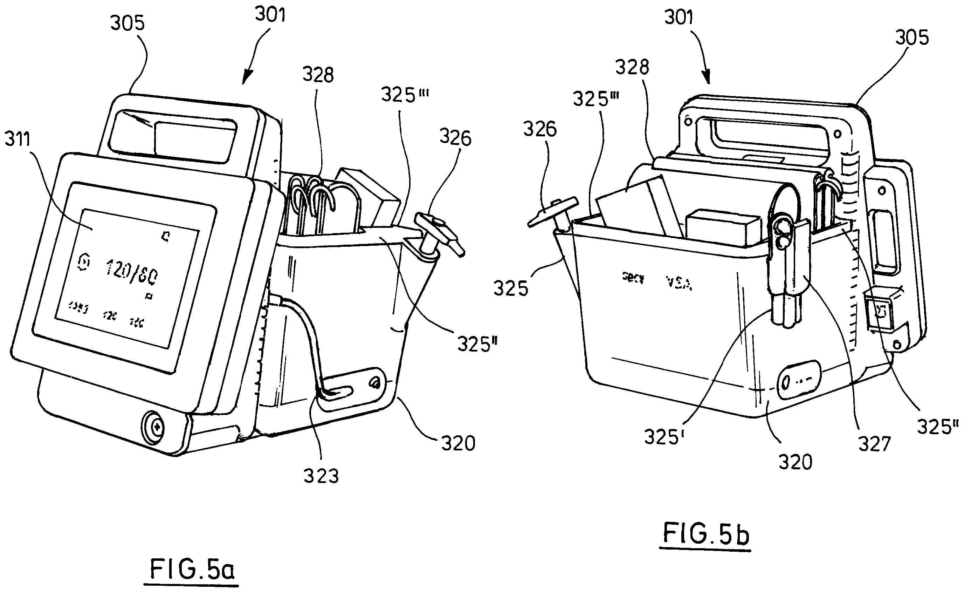

Figur 5a zeigt ein Messsystem 301 in einer Vorderansicht. Es sind eine Steuereinheit 305 mit grafischer Benutzerschnittstelle 311 sowie eine Sensoreinheit 320 erkennbar. Die Sensoreinheit 320 ist über ein USB-Kabel 323 mit der Steuereinheit verbunden. Figur 5b zeigt das Messsystem 301 in einer Rückansicht.

Das Messsystem 301 ist mit einem Sensor 326 zur Körpertemperaturmessung, einem Sensor 327 zur Messung der kapillaren Sauerstoffsättigung und mit einem Sensor 328 zur Blutdruckmessung konfiguriert. In der Sensoreinheit 320 sind mechanische Aufnahmen 325, 325', 325" für die Sensoren 326, 327, 328 vorgesehen. Weiterhin ist ein Staufach 325"' vorgesehen, in welchem Bedienungsanleitungen und Zubehör für die Sensoren 326, 327, 328 vorgehalten werden können.

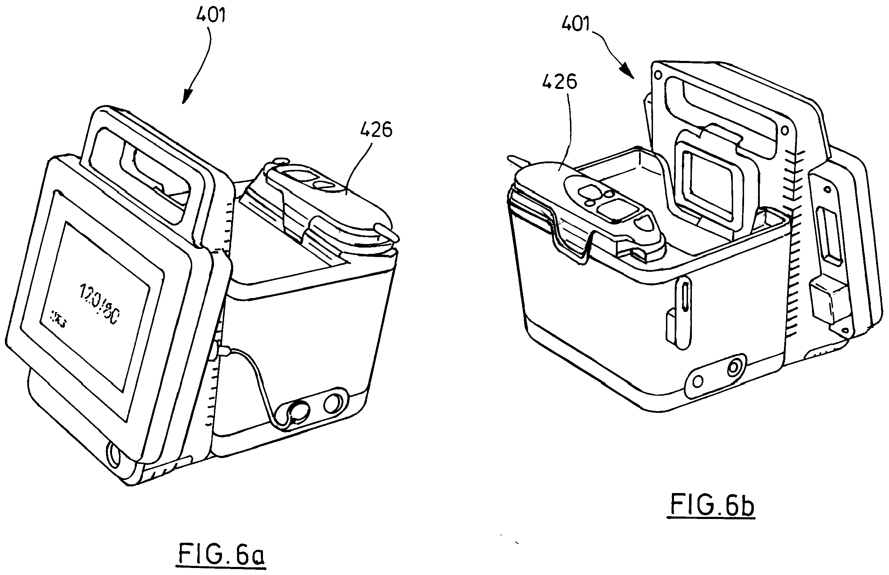

Bei dem Sensor 326 des Messsystems 301 handelt es sich um eine Rektalsonde. In den Figuren 6a und 6b ist ein alternatives Messsystem 401 dargestellt, welches stattdessen mit einem Sensor 426 zur Temperaturmessung ausgeführt ist, bei dem es sich um ein IR-Thermometer zur Messung im Ohr handelt. Die anderen Sensoren des Messsystems 401 entsprechen den Sensoren des Messsystems 301 und sind der Übersichtlichkeit halber nicht dargestellt.

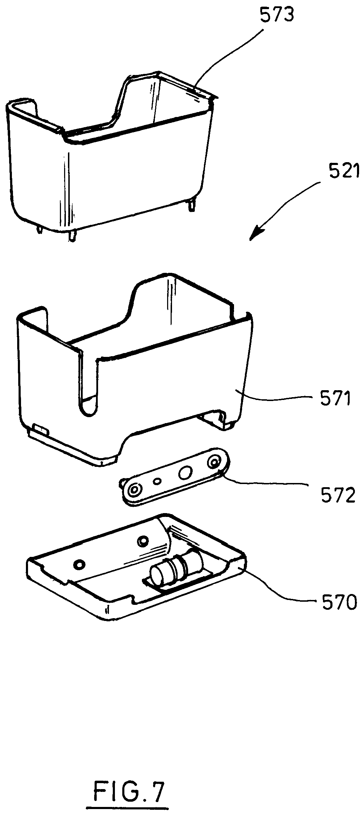

Figur 7 zeigt ein Sensorgehäuse 521 in einer Explosionsdarstellung. Das Sensorgehäuse 521 umfasst einen unteren Abschnitt 570, in welchem nicht dargestellte Schaltungselemente wie die Sensorverbindungseinheit untergebracht sind. Der untere Abschnitt 570 ist mit einem Gehäuserahmen 571 verbunden. Zwischen dem Gehäuserahmen 571 und dem unteren Abschnitt 570 ist eine Verbindungsleiste 572 angeordnet, in welcher elektrische und/oder pneumatische Anschlüsse für Sensoren vorgesehen sind. In den Gehäuserahmen 571 ist von oben ein Aufnahmeabschnitt 573 eingesetzt, in welchem mechanische Aufnahmen für nicht dargestellte Sensoren und gegebenenfalls ein Staufach für Zusatzmaterial vorgesehen sind.

Durch das vorstehend bereits im Detail erläuterte Gerätekonzept können die Funktionen eines mBcA sowie einer Messung von Vitalparametern zusammengeführt werden. Gemäß einer bevorzugten Ausführungsform besteht beim modularen Geräteaufbau eine Grundausstattung aus der Zentraleinheit mit Bildschirm und einer Messmatte für die BIA-Messung. An einer Rückseite des Gerätes kann ein Zusatzmodul angekoppelt werden, dass beispielsweise für die Blutdruckmessung eine Manschette und/oder einen Fingerklipp zur Erfassung der Sauerstoffsättigung aufweist.

Über die bereits erläuterte Koppelung der Zentraleinheit mit den Zusatzmodulen ist es insbesondere möglich, dass die Zentraleinheit die angeschlossenen Komponenten automatisch erkennt und gegebenenfalls konfiguriert.

## Patentansprüche

1. Messeinrichtung zur Bestimmung physiologischer Messwerte eines Patienten mit
- einer grafischen Benutzerschnittstelle,
- einer Steuer- und Auswerteeinheit,
- einer Sensorverbindungseinheit, sowie
- einem oder mehreren Sensoren zur Aufnahme vorbestimmter physiologischer Messwerte,
wobei die Sensoren mit der Sensorverbindungseinheit mittels separater serieller Sensorschnittstellen verbunden sind, und die Sensorverbindungseinheit mit der Steuer- und Auswerteeinheit mittels einer seriellen Steuerungsschnittstelle verbunden ist,
**dadurch gekennzeichnet, dass** eine oder mehrere der Sensorschnittstellen als galvanische getrennte Schnittstellen sein können.

2. Messeinrichtung nach Anspruch 1, **dadurch gekennzeichnet, dass** die Sensorschnittstellen als RS232-Schnittstellen ausgeführt sind.

3. Messeinrichtung nach Anspruch 1 oder 2, **dadurch gekennzeichnet, dass** die Steuerungsschnittstelle als USB-Schnittstelle ausgeführt ist.

4. Messeinrichtung nach einem der Ansprüche 1 bis 3, **dadurch gekennzeichnet**, das eine oder mehrere der Sensorschnittstellen als galvanisch nicht getrennte Schnittstellen ausgeführt sind.

5. Messeinrichtung nach einem der Ansprüche 1 bis 4, **dadurch gekennzeichnet, dass** die Steuer- und Auswerteeinheit wenigstens eine Schnittstelle zum direkten Anschluss weiterer Sensoren aufweist.

6. Messeinrichtung nach einem der Ansprüche 1 bis 5, **dadurch gekennzeichnet, dass** die grafische Benutzerschnittstelle und die Steuer- und Auswerteeinheit in einem Steuerungsgehäuse angeordnet sind, und dass die Sensorverbindungseinheit in einem Sensorgehäuse angeordnet ist.

7. Messeinrichtung nach Anspruch 6, **dadurch gekennzeichnet, dass** das Steuerungsgehäuse und/oder das Sensorgehäuse Verbindungsmittel aufweisen, um die Gehäuse lösbar miteinander zu verbinden, und dass die Verbindung zwischen der Steuer- und Auswerteeinheit und der Sensorverbindungseinheit als Steckverbindung ausgeführt ist.

8. Messeinrichtung nach Anspruch 6 oder 7, **dadurch gekennzeichnet, dass** das Sensorgehäuse mechanische Aufnahmemittel zur Aufnahme eines oder mehrerer Sensoren aufweist.

9. Messeinrichtung nach Anspruch 8, **dadurch gekennzeichnet, dass** die mechanischen Aufnahmemittel zur Aufnahme spezifischer Sensortypen ausgeführt sind, und dass die Sensorverbindungseinheit zur Kommunikation mit denselben spezifischen Sensortypen konfiguriert ist.

10. Messeinrichtung nach Anspruch 9, **dadurch gekennzeichnet, dass** die Steuer- und Auswerteeinheit eingerichtet ist, die Konfiguration der Sensorverbindungseinheit zu ermitteln und die grafische Benutzerschnittstelle so anzusteuern, dass nur mit der vorliegenden Konfiguration kompatible Eingaben und/oder Ausgaben möglich sind.

11. Messeinrichtung nach Anspruch 9, **dadurch gekennzeichnet, dass** das Messsystem als modulares Messsystem ausgeführt ist, welches
- eine erste Sensoreinheit mit einer ersten Sensorverbindungseinheit in einem ersten Sensorgehäuse und eine erste Gruppe von Sensoren, sowie
- eine zweite Sensoreinheit mit einer zweiten Sensorverbindungseinheit in einem zweiten Sensorgehäuse und eine zweiten Gruppe von Sensoren umfasst,
wobei die Benutzerschnittstelle und die Steuer- und Auswerteeinheit wahlweise mit der ersten Sensoreinheit oder mit der zweiten Sensoreinheit zu einem Messsystem verbunden werden kann.

12. Sensoreinheit einer Messeinrichtung gemäß Anspruch 10.
